(19) Europäisches Patentamt
European Patent Office
Office européen des brevets

(11) **EP 0 769 687 B1**

(12) **EUROPÄISCHE PATENTSCHRIFT**

(45) Veröffentlichungstag und Bekanntmachung des Hinweises auf die Patenterteilung:
**05.12.2001 Patentblatt 2001/49**

(51) Int Cl.⁷: **G01G 17/02**, G01G 3/16, G01N 33/36

(21) Anmeldenummer: **96116407.6**

(22) Anmeldetag: **14.10.1996**

(54) **Verfahren zur Bestimmung der Masse pro Längeneinheit von natürlichen oder synthetischen Fäden oder Fasern und Vorrichtung zur Durchführung des Verfahrens**

Method for determining the mass per unit length for natural or synthetic filaments or fibres and device for carrying out this method

Méthode de détermination de la masse par unité de longueur de filaments ou de fibres naturelles ou synthétiques et dispositif pour la réalisation de ce procédé

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **18.10.1995 AT 173895**

(43) Veröffentlichungstag der Anmeldung:
**23.04.1997 Patentblatt 1997/17**

(73) Patentinhaber: **LENZING AKTIENGESELLSCHAFT**
**4860 Lenzing (AT)**

(72) Erfinder: **Ramsauer, Christoph Norbert**
**5360 St. Wolfgang (AT)**

(74) Vertreter: **Kopecky, Helmut, Dipl.-Ing. et al**
**Kopecky & Schwarz**
**Patentanwälte**
**Wipplingerstrasse 32/22**
**1010 Wien (AT)**

(56) Entgegenhaltungen:
**EP-A- 0 610 147**        **DE-A- 1 448 061**
**DE-A- 2 048 852**        **DE-A- 2 925 801**
**GB-A- 2 020 816**

Bemerkungen:
Die Akte enthält technische Angaben, die nach dem Eingang der Anmeldung eingereicht wurden und die nicht in dieser Patentschrift enthalten sind.

## Beschreibung

Die Erfindung betrifft ein Verfahren zur Bestimmung der Masse pro Längeneinheit (der Titer) von natürlichen oder synthetischen Fäden oder Fasern sowie eine Vorrichtung zu Durchführung des Verfahrens.

Es ist bekannt, aus der Eigenfrequenz einer zugbelasteten Faser bei bekannter Einspannlänge und Faserspannung den Titer zu errechnen. Dieses Verfahren wird als "Schwingungsmethode" bezeichnet. Der Begriff "Schwingungsmethode" besagt, daß eine zu prüfende Faser durch eine geeignete Methode in Eigenschwingung versetzt wird und daraus ihre lineare Massendichte ermittelt wird. Die Methode macht sich die Beziehung

$$T = konst* \frac{p}{l^2 * f^2}$$

zunutze. Dabei bedeuten

T    den Titer
p    die Belastung des eingespannten Fadens
l    die schwingende Fadenlänge im Resonanzfall und
f    die zugehörige Resonanzfrequenz.

Hat man also bei bekannter Einspannlänge sowie Belastung des eingespannten Fadens die Resonanzfrequenz ermittelt, läßt sich daraus unmittelbar der Titer des Fadens bestimmen.

Bisher wurden zwei Methoden angewandt, um die Eigenfrequenz des eingespannten Fadens zu ermitteln:

Bei einer bekannten Methode wurde die Einspannlänge des Fadens so lange verändert, bis die Resonanzlänge des Fadens erreicht war und somit die Eigenschwingung auftrat. Eine solche Methode ist z.B. aus der AT-B 300418 bekannt. Diese Methode ist relativ aufwendig und mit einem gewissen Fehler behaftet.

Bei der zweiten bekannten Methode wird bei konstanter Einspannlänge des Fadens die Frequenz des Schwingungserregers so lange verändert, bis ebenfalls die Resonanzbedingung erfüllt ist. Die konstante Einspannlänge und die Variation der Frequenz vereinfachen zwar die Messung, die Messung ist aber mit einem schlecht zu schätzenden Phasenfehler behaftet. Außerdem leidet die Stabilität des Schwingungsmodus.

Die vorliegende Erfindung stellt sich die Aufgabe, ein Verfahren sowie eine Vorrichtung zur Durchführung des Verfahrens zur Verfügung zu stellen, mit denen die oben erwähnten Nachteile des Standes der Technik überwunden werden können und somit in einfacher Weise eine präzise Bestimmung des Titers von Fäden bzw. Fasern ermöglicht wird.

Die Aufgabe der Erfindung wird durch ein Verfahren zur Bestimmung der Masse pro Längeneinheit (des Titers) von natürlichen oder synthetischen Fäden oder Fasern, wobei ein unter Zugkraft stehender Einzelfaden zu einer Schwingung in Eigenfrequenz angeregt wird und aus der Eigenfrequenz die Masse pro Längeneinheit ermittelt wird, gelöst, welches dadurch gekennzeichnet ist, daß dem Faden berührungslos ein Stoß in Form eines elektrischen Deltaimpulses versetzt wird.

Ein Deltaimpuls wird durch das schnelle Ein- und Ausschalten eines Generators erzeugt. Wenn der Generator beim kontinuierlichen Betrieb eine bestimmte Frequenz erzeugt, so wird in der kurzen Zeitdauer zwischen Ein- und Ausschalten ein ganzes Frequenzband rund um diese Grundfrequenz erzeugt. Der Name "Deltaimpuls" leitet sich vom Verlauf der Kurve der Impulsstärke gegen die Zeitspanne (Pulslänge) des Impulses ab. Je kürzer diese Zeitspanne ist, umso breiter ist das erzeugte Frequenzband. Theoretisch kann somit ein Deltaimpuls eine unendliche Bandbreite besitzen.

Da innerhalb dieser großen Bandbreite auch die Eigenfrequenz des eingespannten Fadens enthalten ist, wird der Faden, wenn ihm berührungslos ein Stoß in Form eines elektrischen Deltaimpulses versetzt wird, automatisch zu einer gedämpften Schwingung in der Eigenfrequenz angeregt, ohne daß diese Schwingung von weiteren Frequenzen überlagert wäre. Der Schwingungsvorgang schwingt exponentiell ab.

Die Eigenschwingung des eingespannten Fadens wird somit in Bruchteilen von Sekunden erreicht, ohne daß in aufwendigen Verfahren die Einspannlänge oder die Erregerfrequenz zum Auffinden der Eigenfrequenz variiert werden müssen.

Die Eigenschwingung wird sofort in ein elektrisches Signal umgewandelt. Aus diesem Signal kann durch bekannte numerische sowie approximative Verfahren die Frequenz der Eigenschwingung ermittelt werden.

Die Stärke der Einkopplung des Deltaimpulses kann in einem weiten Bereich durch ein elektrisches Gleichfeld in Richtung der Faser geregelt werden. Dadurch lassen sich sowohl gut als auch schlecht elektrisch leitende Fasern prüfen. Durch das Arbeiten mit einer feststehenden Einspannlänge sind auch kurze Faserstücke, z.B. kurz geschnittene Stapelfasern prüfbar. Solche Fasern ließen sich mit den Methoden des Standes der Technik bis jetzt nur sehr schwer oder gar nicht prüfen.

Vorzugsweise weist der Deltaimpuls eine Spannung von 5 kV - 8 kV auf. Die Pulslänge des Deltaimpulses beträgt in vorteilhafter Weise 200μs bis 300μs, vorzugsweise 250μs bis 280μs.

In weiters bevorzugter Weise ist das erfindungsgemäße Verfahren dahingehend ausgestaltet, daß die für das Anschwingen der Faser notwendige Ladung mittels einer Gleichspannung von 600 V bis 1200V zugeführt wird.

Die mechanische Schwingung des Fadens wird in besonders vorteilhafter Weise mittels einer Photodiode mit gekrümmtem Rand in ein elektrisches Signal umgewandelt.

Dabei wird das reelle Bild des zu prüfenden Fadens auf die Photodiode abgebildet. Wenn sich der Faden in Schwingung befindet, erzeugt sein Schatten ein dem Gleichlichtanteil aufmoduliertes elektrisches Wechselsignal exakt gleicher Frequenz. Die Phasenlage spielt dabei für die nachfolgende Signalbearbeitung keine Rolle. Das elektrische Signal kann z.B. mit einem A/D-Wandler mit zumindest 300 Stützpunkten pro Periode digitalisiert sowie einem digitalen Bandpaßfilter unterworfen werden. Aus dem auf diese Weise aufbereiteten Digitalsignal kann wie oben erwähnt durch verschiedene numerische sowie approximative Verfahren die Frequenz der Eigenschwingung ermittelt werden.

Zur Durchführung des erfindungsgemäßen Verfahrens dient eine Vorrichtung mit einer Einspannvorrichtung zur Einspannung eines Fadens unter Zug, einem Schwingungserreger und einer Meßvorrichtung zur Ermittlung der Eigenfrequenz des Fadens, welche dadurch gekennzeichnet ist, daß der Schwingungserreger in Form eines Plattenkondensators ausgebildet ist, der den zu prüfenden Faden umgibt.

Durch den Plattenkondensator, welcher den zu prüfenden Faden umgibt, wird der Deltaimpuls zur Anregung der Eigenschwingung des Fadens zugeführt.

Die Meßvorrichtung beinhaltet vorzugsweise eine Photodiode mit gekrümmtem Rand sowie eine Auswertungseinheit zur Ermittlung der Eigenfrequenz aus dem durch die Photodiode erzeugten elektrischen Signal.

Die Erfindung ist in den Zeichnungen an einem Ausführungsbeispiel näher erläutert. Fig. 1 zeigt eine Ausführungsform der Vorrichtung zur Durchführung des erfindungsgemäßen Verfahrens in Vorderansicht, Fig. 2 in Draufsicht und Fig. 3 zeigt schematisch das Meßprinzip der erfindungsgemäß vorzugsweise eingesetzten Photodiode mit gekrümmtem Rand zur Umwandlung der Eigenschwingung in ein elektrisches Signal.

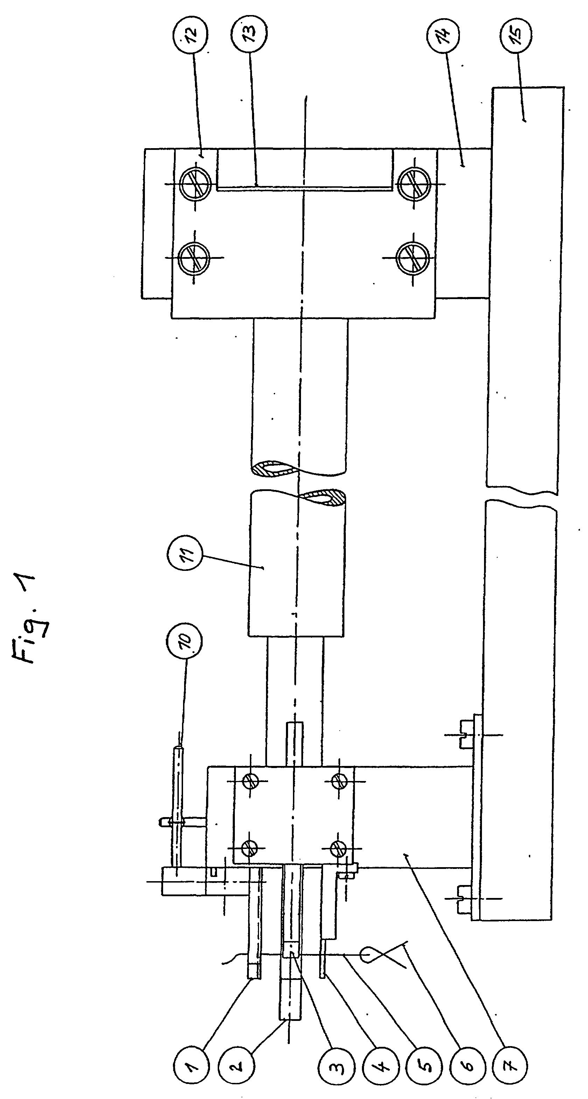

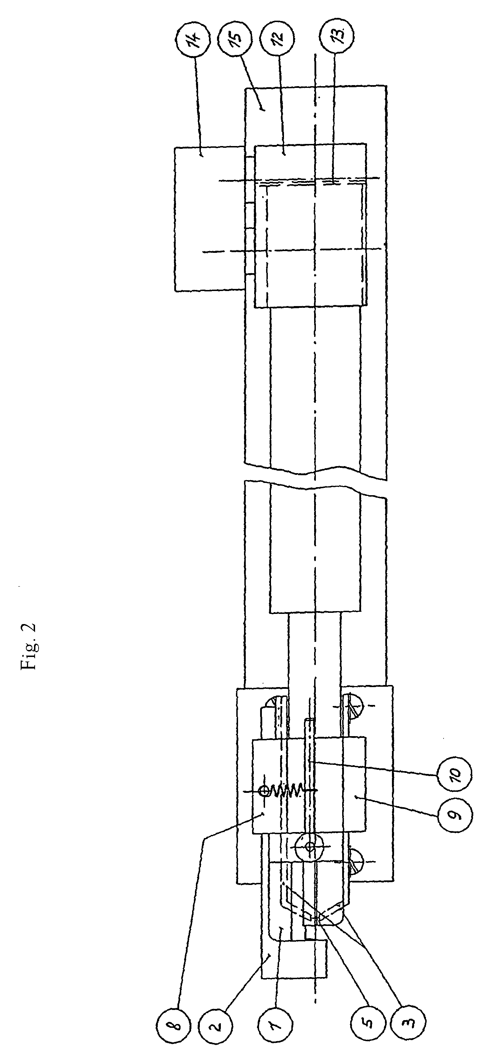

Eine Ausführungsform der Vorrichtung zur Durchführung des erfindungsgemäßen Verfahrens weist gemäß Fig. 1 und Fig. 2 Baugruppenträger 7, 14, 15 auf. Der zu prüfende Faden 5 wird zwischen eine Klemmvorrichtung 1 und ein Belastungsgewicht 6 eingespannt und somit auf Zug belastet. Die Klemme wird mittels eines gefederten Hebels 10 betätigt. Der Faden wird durch zwei Wechselspannungselektroden 3 durchgeführt, welche durch Halterungen 8, 9 am Baugruppenträger 7 befestigt sind.

Zur Prüfung der Faser wird diese einem Deltaimpuls mit einer Pulslänge von beispielsweise 260µs ausgesetzt. Das reelle Bild der mit Eigenfrequenz schwingenden Faser wird durch eine Beleuchtungsvorrichtung 2 über einen Lichtstrahlkanal 11 auf eine in einer Halterung 12 vorgesehene Photozelle 13 abgebildet.

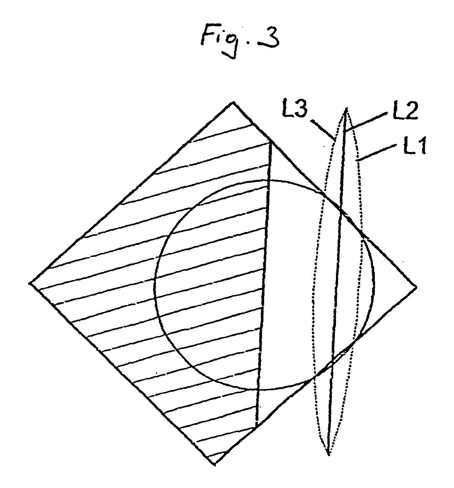

Die Wirkung der Photodiode 13 ist in der Fig. 3 schematisch angedeutet. Das reelle Bild der zu prüfenden Faser (mit L2 bezeichnet) wird wie in der Abbildung gezeigt auf eine Photodiode abgebildet. Wenn sich die Faser in Schwingung befindet (durch die Endpositionen L1 und L3 angedeutet), erzeugt ihr Schatten ein dem Gleichlichtanteil aufmoduliertes elektrisches Wechselsignal exakt gleicher Frequenz. Das elektrische Signal wird mit einem schnellen A/D-Wandler (vorzugsweise über 300 Stützpunkte pro Periode) digitalisiert sowie einem digitalen Bandpaßfilter unterworfen. Aus dem auf diese Weise aufbereiteten Digitalsignal kann z.B. durch eine Fouriertransformation die Eigenfrequenz und daraus der Titer berechnet werden.

Es zeigt sich, daß bei der Durchführung des erfindungsgemäßen Verfahrens sowie bei der Verwendung der zur Durchführung des Verfahrens geeigneten erfindungsgemäßen Vorrichtung die Prüfgeschwindigkeit im Vergleich zum Stand der Technik drastisch erhöht wird. Aufgrund der Möglichkeit einer automatischen Durchführung des Verfahrens ist die Gefahr subjektiver Meßfehler minimiert. Der Meßfehler ist im Vergleich zum Stand der Technik sehr klein. Zudem erweist sich die überraschend einfache Konstruktion von zur Durchführung des erfindungsgemäßen Verfahrens geeigneten Vorrichtungen als preisgünstig im Vergleich zu komplizierteren Vorrichtungen des Standes der Technik.

**Patentansprüche**

1. Verfahren zur Bestimmung der Masse pro Längeneinheit (des Titers) von natürlichen oder synthetischen Fäden oder Fasern, wobei ein unter Zugkraft stehender Einzelfaden zu einer Schwingung in Eigenfrequenz angeregt wird und aus der Eigenfrequenz die Masse pro Längeneinheit ermittelt wird, **dadurch gekennzeichnet, daß** dem Faden berührungslos ein Stoß in Form eines elektrischen Deltaimpulses versetzt wird.

2. Verfahren nach Anspruch 1, **dadurch gekennzeichnet, daß** der Deltaimpuls eine Spannung von 5 kV - 8 kV aufweist.

3. Verfahren nach einem der Ansprüche 1 oder 2, **dadurch gekennzeichnet, daß** die für das Anschwingen der Faser notwendige Ladung mittels einer Gleichspannung von 600 V bis 1200V zugeführt wird.

4. Verfahren nach einem der vorherigen Ansprüche, **dadurch gekennzeichnet, daß** die mechanische Schwingung des Fadens mittels einer Photodiode mit gekrümmtem Rand in ein elektrisches Signal umgewandelt wird.

5. Vorrichtung zur Durchführung des Verfahrens nach einem der Ansprüche 1 bis 3 mit einer Einspannvorrichtung (1, 4, 6) zur Einspannung eines Fadens unter Zug, einem Schwingungserreger (3) und einer Meßvorrichtung (13) zur Ermittlung der Eigenfrequenz des Fadens, **dadurch gekennzeichnet, daß** der Schwingungserreger in Form eines Plattenkondensators (3) ausgebildet ist, der den zu prüfenden Faden (5) umgibt.

6. Vorrichtung nach Anspruch 5, **dadurch gekennzeichnet, daß** die Meßvorrichtung eine Photodiode mit gekrümmtem Rand sowie eine Auswertungseinheit zur Ermittlung der Eigenfrequenz aus dem durch die Photodiode erzeugten elektrischen Signal beinhaltet.

**Claims**

1. Method for determining the mass per unit length (the titre) of natural or synthetic threads or fibres, in which a single thread, subjected to a tensile force, is excited into oscillation at its natural frequency and the mass per unit length is determined from the natural frequency, **characterised in that** an impulse in the form of an electrical delta pulse is transferred, without contact, to the thread.

2. Method according to Claim 1, **characterised in that** the delta pulse exhibits a voltage of 5 kV - 8 kV.

3. Method according to one of Claims 1 and 2, **characterised in that** the charge required to set the fibre into oscillation is delivered in the form of a DC voltage of 600 V to 1200 V.

4. Method according to one of the preceding claims, **characterised in that** the mechanical oscillation of the thread is converted into an electrical signal by means of a photodiode with a rounded edge.

5. Device for carrying out the method according to one of Claims 1 to 3, with a workpiece-holding device (1, 4, 6) for holding a thread under tension, an oscillation generator (3) and a measuring device (13) for determining the natural frequency of the thread, **characterised in that** the oscillation generator is implemented in the form of a plate capacitor (3) which surrounds the thread (5) to be tested.

6. Device according to Claim 5, **characterised in that** the measuring device includes a photodiode with a rounded edge as well as an evaluation unit for determining the natural frequency from the electrical signal produced by the photodiode.

**Revendications**

1. Procédé pour déterminer la masse par unité de longueur (le titre) de fils ou de fibres naturels ou synthétiques, dans lequel on excite un fil individuel disposé sous une force de traction en oscillations à la fréquence propre, et on détermine à partir de la fréquence propre la masse par unité de longueur, **caractérisé en ce que** l'on applique au fil sans contact un choc sous la forme d'une impulsion électrique delta.

2. Procédé selon la revendication 1, **caractérisé en ce que** l'impulsion delta présente une tension de 5 kV à 8 kV.

3. Procédé selon l'une ou l'autre des revendications 1 et 2, **caractérisé en ce que** la charge nécessaire pour la mise des fibres en oscillations est apportée au moyen d'une tension continue de 600 V à 1200 vols.

4. Procédé selon l'une des revendications précédentes, **caractérisé en ce que** l'oscillation mécanique du fil est convertie en un signal électrique avec une photodiode à bord incurvé.

5. Appareil pour la mise en oeuvre du procédé selon l'une des revendications 1 à 3, comprenant un dispositif de serrage (1, 4, 6) pour mettre un fil sous tension, un excitateur d'oscillations (3), et un dispositif de mesure (13) pour déterminer la fréquence propre du fil, **caractérisé en ce que** l'excitateur d'oscillations est réalisé sous la forme d'un condensateur à plaques (3) qui entoure le fil à tester (5).

6. Appareil selon la revendication 5, **caractérisé en ce que** le dispositif de mesure comprend une photodiode avec bord incurvé, ainsi qu'une unité d'évaluation pour déterminer la fréquence propre à partir du signal électrique produit par la photodiode.

Fig. 1

Fig. 2

EP 0 769 687 B1

Fig. 3